Europäisches Patentamt

European Patent Office

Office européen des brevets

(19)

(11) Publication number: **0 058 489**
**B1**

(12) **EUROPEAN PATENT SPECIFICATION**

(45) Date of publication of patent specification: **12.09.84**

(21) Application number: **82300527.7**

(22) Date of filing: **02.02.82**

(51) Int. Cl.³: **A 61 F 1/00,** A 61 G 7/04, B 32 B 19/06

(54) Prosthetic devices for pressure sores.

(30) Priority: **11.02.81 GB 8104211**

(43) Date of publication of application:
**25.08.82 Bulletin 82/34**

(45) Publication of the grant of the patent:
**12.09.84 Bulletin 84/37**

(84) Designated Contracting States:
**AT BE CH DE FR IT LI NL SE**

(56) References cited:
**EP-A-0 009 072
FR-A-1 440 008
FR-A-2 308 843
GB-A-1 600 768
US-A-2 410 884
US-A-3 850 785**

(73) Proprietor: **DUNLOP LIMITED**
**Dunlop House Ryder Street St. James's
London SW1Y 6PX (GB)**

(72) Inventor: **Sherrin, Alan John
5 Holte Drive Four Oaks
Sutton Coldfield West Midlands (GB)**
Inventor: **Higgs, Maurice William
175 Foley Road West, Streetly
Sutton Coldfield West Midlands (GB)**

(74) Representative: **Waller, Roy Ernest Sykes et al
Group Patent Department Dunlop Limited 2
Parade
Sutton Coldfield West Midlands B72 1PF (GB)**

Courier Press, Leamington Spa, England.

## Description

This invention relates to a prosthetic device for use in the treatment or prevention of pressure sores.

In the treatment of pressure sores in which there is exhibited attack and erosion of the body tissue which normally underlies the skin, generally known as third degree pressure sores, it is the practice often to effect treatment by drawing over the affected area the surrounding skin. This form or treatment is not, however, entirely satisfactory insofar as further pressure sores tend soon to develop again at or near the treated area.

As an alternative method of treatment, and with the hope of reducing the tendency for further sores to develop at or near the same area, use has been made of silicone rubber implants. These have not, however, been entirely satisfactory because they are not easily fixed within the body and can migrate after implantation. Also insufficient vascularisation beneath the new skin flap increases the chance of further breakdown.

The present invention has as one of its objects the provision of a prosthetic device suitable for use in the treatment and prevention of pressure sores and in which the aforementioned problems are mitigated or overcome.

In accordance with the present invention there is provided a prosthetic device for the treatment and prevention of pressure sores comprising a resilient pad of a sandwich type construction and formed from carbon fibre material, said pad comprising a first cushion layer of carbon fibre material which is sandwiched between, and relatively resilient in comparison with, a pair of support layers also of carbon fibre material.

The resiliency of the cushion layer material preferably is substantially greater than that of the support layer material. Preferably it is in the range two to five times that of the material of the support layers.

The support layers, although of low resiliency material, preferably are sufficiently flexible so as to readily deform to the shape of a surface against which they are placed in contact, said deformation being readily accommodated by the relatively high resiliency of the cushion layer. Preferably the flexibility of the support layer as measured in terms of percentage compression under a load of 300 gms/cm² is at least 15%.

The support layers preferably each have a thickness no more than 50% and no less than 10% of the thickness of the resilient cushion layer so that the support layers each exhibit a suitable overall flexibility.

Although being of low flex resistance, the support layer material preferably is of a relatively high tensile strength as compared with that of the cushion layer material so as to facilitate handling of the pad during surgical operations and subsequent maintenance of the required overall general shape during use.

Preferably the tensile strength of the material forming the support layers is greater than 5N/cm, typically in the order of 9N/cm on a stitch strength tear test. When fully embedded in connective tissue a material which per se has a strength of 9N/cm results in an effective strength of 63N/cm.

Suitable types of constructions for forming the support layers include woven fabric, knitted fabric, aligned mat, stitch bonded fabric and lace fabric, in each case the fabric or mat being formed from the carbon fibre material.

Suitable constructions for forming the resilient cushion layer include bat or non-woven staple fibre arrangements, and spin bonded continuous filament type arrangements.

A convenient method of securing together the adjacent layers is the use of a needle punching technique.

Carbonisation of the materials preferably is effected subsequent to formation and assembly of the layers forming the prosthesis, the materials being easier to weave and otherwise handle prior to carbonisation.

Suitable materials for forming the support and cushion layers include oxidised polyacrylonitrile fibre, cellulose, rayon, pitch, polyimide or polyvinyl alcohol fibre. The first referred of these materials is preferred for most uses.

Particularly in the case of layers formed from oxidised polyacrylonitrile fibres, carbonisation of the assembled layers may be effected in an atmosphere of pure nitrogen at a temperature of at least 800°C, typically 1,000°C. Subsequent to carbonisation the prosthesis so formed is cleaned, sterilised and packaged for use.

One or each of the support layers between which the cushion layer is interposed may form a surface layer of the prosthesis or be covered by one or more further layers, preferably of carbonised material. Additionally, one or more support layers may be provided within the cushion layer to confer increased stability to the overall shape of the prosthesis, particularly in constructions in which the cushion layer is of relatively great overall thickness so as to provide a high degree of resiliency.

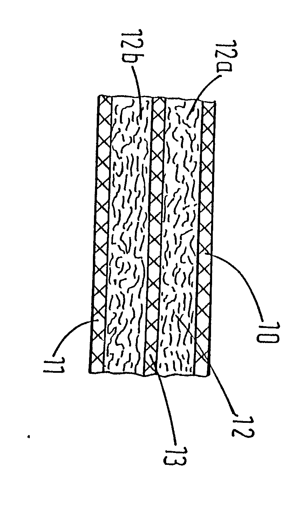

One embodiment of the invention will now be described, by way of example, with reference to the accompanying drawing which is a cross-sectional view of a prosthesis in accordance with the present invention.

A prosthesis for the treatment of pressure sores is in the form of a multi-layered resilient pad and comprises a pair of surface layers 10, 11 having sandwiched therebetween a cushion layer 12. The cushion layer is formed by two sub-layers 12a, 12b which in turn have sandwiched therebetween a support layer 13 of similar construction to the surface layers 10, 11. The pad has an overall thickness of 1.9 cm ($\frac{3}{4}$"), and the support and surface layers each have a thickness in the order of 0.16 cm (1/16").

The surface, internal support and cushion layers are each formed from oxidised polyacrylonitrile fibre. The internal support and the surface layers are formed from a woven fabric of said fibres, and the cushion sub-layers 12a, 12b are each formed from a non-woven stable bat of said fibres.

The five layers are secured together by needle punching, and subsequently carbonised in an atmosphere of pure nitrogen at 1,000°C.

In use of the prosthesis for implanting in the treatment of pressure sores the surface and central support layers 10, 11, 13 assist in maintaining integrity to the shape of the prosthesis, and also provide anchorage for surgical sutures. Subsequent to implantation body tissue relatively readily grows and penetrates around the carbon fibre material of the surface layers to result in firm retention and transmission of load between the overlying skin, the prosthesis and underlying body tissues. The central support layer assists in maintaining the overall shape of the prosthesis despite the relatively high resiliency of the cushion layer material which is thereby able to accommodate localised deformation and uniformly distribute load.

## Claims

1. Prosthetic device for the treatment and prevention of pressure sores characterised in that it comprises a resilient pad of a sandwich type construction and formed from carbon fibre material, said pad comprising a first cushion layer (12) of carbon fibre material which is sandwiched between, and relatively resilient in comparison with, a pair of support layers (10, 11) also of carbon fibre material.

2. Prosthetic device according to Claim 1 characterised in that the resiliency of the cushion layer material (12) is substantially greater than the resilience of the support layer material.

3. Prosthetic device according to Claim 2 characterised in that the resiliency of the cushion layer material is in the range 2 to 5 times that of the material of the support layers.

4. Prosthetic device according to any one of the Claims 1 to 3 characterised in that at least one additional support layer (13) is provided within the cushion layer (12).

5. Prosthetic device according to any one of the preceding claims characterised in that the support layers (10, 11) each have a thickness in the range 10% to 50% of the thickness of the resilient cushion layer.

6. Prosthetic device according to any one of the preceding claims characterised in that the flexibility of the support layers (10, 11) as measured in terms of percentage compression under a load of 300 gms/cm² is at least 15%.

7. Prosthetic device according to any one of the preceding claims characterised in that the support layer material is of a relatively high tensile strength as compared with that of the cushion layer material.

8. Prosthetic device according to Claim 7 characterised in that the tensile strength of the material forming the support layers is greater than 5N/cm as measured on a stitch strength tear test.

9. Prosthetic device according to any one of the preceding claims characterised in that adjacent layers have been secured together by needle punching.

10. Prosthetic device according to any one of the preceding claims characterised in that at least one of the support layers (10, 11) forms a surface layer of the prosthesis.

11. Prosthetic device according to any one of Claims 1 to 9 characterised in that a support layer is covered by one or more further layers.

12. Prosthetic device according to any one of the preceding claims characterised in that carbonisation of the materials forming the device has been effected subsequent to formation and assembly of the layers.

13. Prosthetic device according to any one of the preceding claims characterised in that a support and/or cushion layer is formed of oxidised polyacrylonitrile fibre.

## Patentansprüche

1. Prothetische Einrichtung zur Behandlung und Vermeidung von Druckwunden, dadurch gekennzeichnet, daß sie ein elastisches Kissen mit einem sandwichartigen Aufbau aus einem Kohlefasermaterial umfaßt, welches Kissen eine erste Polsterschicht (12) aus Kohlefasermaterial umfaßt, die in Sandwich-Bauweise zwischen einem Paar von Stützschichten (10 und 11) gleichfalls aus Kohlefasermaterial angeordnet ist und im Vergleich mit diesen relativ elastisch ist.

2. Prothetische Einrichtung nach Anspruch 1, dadurch gekennzeichnet, daß die Elastizität des Polsterschichtmaterials (12) wesentliche größer als die Elastizität des Stützschichtmaterials ist.

3. Prothetische Einrichtung nach Anspruch 2, dadurch gekennzeichnet, daß die Elastizität des Polsterschichtmaterials im Bereich des Zweifachen bis Fünffachen der des Materials der Stützschichten liegt.

4. Prothetische Einrichtung nach einem der Ansprüche 1 bis 3, dadurch gekennzeichnet, daß wenigstens eine zustätzliche Stützschicht (13) in der Polsterschicht (12) vorgesehen ist.

5. Prothetische Einrichtung nach einem der vorhergehenden Ansprüche, dadurch gekennzeichnet, daß die Stützchichten (10 und 11) jeweils eine Stärke im Bereich von 10 bis 50% der Stärke der elastischen Polsterschicht haben.

6. Prothetische Einrichtung nach einem der vorhergehenden Ansprüche, dadurch gekennzeichnet, daß die Biegsamkeit der Stützschichten (10, 11) gemessen in Prozent Kompression unter einer Belastung von 300 g/cm² wenigstens 15% beträgt.

7. Prothetische Einrichtung nach einem der vorhergehenden Ansprüche, dadurch gekennzeichnet, daß das Stützschichtmaterial eine relativ hohe Zugfestigkeit verglichen mit der des Polsterschichtmaterials hat.

8. Prothetische Einrichtung nach Anspruch 7, dadurch gekennzeichnet, daß die Zugfestigkeit des Materials, das die Stützschichten bildet, größer als 5 N/cm gemessen im Stichausreißfestigkeitstest ist.

9. Prothetische Einrichtung nach einem der vorhergehenden Ansprüche, dadurch gekennzeichnet, daß benachbarte Schichten durch Vernadlen aneinander befestigt sind.

10. Prothetische Einrichtung nach einem der vorhergehenden Ansprüche, dadurch gekennzeichnet, daß wenigstens eine der Stützschichten (10, 11) eine Oberflächenschicht der Prothese bildet.

11. Prothetische Einrichtung nach einem der Ansprüche 1 bis 9, dadurch gekennzeichnet, daß eine Stützschicht von einer oder mehreren weiteren Schichten überdeckt ist.

12. Prothetische Einrichtung nach einem der vorhergehenden Ansprüche, dadurch gekennzeichnet, daß die Karbonisierung der Materialien, die die Einrichtung bilden, anschließend an die Ausbildung und die Zusammensetzung der Schichten bewirkt ist.

13. Prothetische Einrichtung nach einem der vorhergehenden Ansprüche, dadurch gekennzeichnet, daß eine Stützschicht und/oder eine Polsterschicht aus oxidierten Polyacrylnitrilfasern besteht.

**Revendications**

1. Dispositif de prothèse pour traiter et empêcher des escarres, caractérisé en ce qu'il comprend un tampon élastique ayant une structure de type sandwich et formé d'une matière à fibres de carbone, ledit tampon comprenant une première couche d'amortissement (12) en matière à fibres de carbone qui est interposée entre, et relativement élastique par comparaison à, une paire de couches de support (10, 11) également en matière à fibres de carbone.

2. Dispositif de prothèse selon la revendication 1, caractérisé en ce que l'élasticité de la matière de couche d'amortissement (12) est sensiblement plus grande que l'élasticité de la matière de couche de support.

3. Dispositif de prothèse selon la revendication 2, caractérisé en ce que l'élasticité de la matière de couche d'amortissement est supérieure de deux à cinq fois à celle de la matière des couches de support.

4. Dispositif de prothèse selon une quelconque des revendications 1 à 3, caractérisé en ce qu'au moins une couche de support additionnelle (13) est placée à l'intérieur de la couche d'amortissement (12).

5. Dispositif de prothèse selon une quelconque des revendications précédentes, caractérisé en ce que les couches de support (10, 11) ont chacune une épaisseur comprise entre 10 et 50% de l'épaisseur de la couche d'amortissement élastique.

6. Dispositif de prothèse selon une quelconque des revendications précédentes, caractérisé en ce que la flexibilité des couches de support (10, 11), mesurée en termes de pourcentage de compression sous une charge de 300 g/cm², est d'au moins 15%.

7. Dispositif de prothèse selon une quelconque des revendications précédentes, caractérisé en ce que la matière de la couche de support a une résistance à la traction relativement grande par comparaison à celle de la matière de couche d'amortissement.

8. Dispositif de prothèse selon la revendication 7, caractérisé en ce que la résistance à la traction de la matière formant les couches de support est supérieure à 5 N/cm, en étant mesurée dans un essai de résistance au déchirement d'une couture.

9. Dispositif de prothèse selon une quelconque des revendications précédentes, caractérisé en ce que des couches adjacentes sont fixées ensemble par aiguilletage.

10. Dispositif de prothèse selon une quelconque des revendications précédentes, caractérisé en ce qu'au moins une des couches de support (10, 11) forme une couche superficielle de la prothèse.

11. Dispositif de prothèse selon une quelconque des revendications 1 à 9, caractérisé en ce qu'une couche de support est recouverte par une ou plusieurs autres couches.

12. Dispositif de prothèse selon une quelconque des revendications précédentes, caractérisé en ce qu'une carbonisation des matières formant le dispositif est effectuée après la formation et l'assemblage des couches.

13. Dispositif de prothèse selon une quelconque des revendications précédentes, caractérisé en ce qu'une couche de support et/ou une couche d'amortissement est formée de fibres de polyacrylonitrile oxydé.